(19) **Europäisches Patentamt / European Patent Office / Office européen des brevets**

(11) **EP 4 171 478 B1**

(12) **EUROPEAN PATENT SPECIFICATION**

(45) Date of publication and mention
of the grant of the patent:
**06.08.2025 Bulletin 2025/32**

(21) Application number: **21731541.5**

(22) Date of filing: **17.06.2021**

(51) International Patent Classification (IPC):
*A61K 8/26* (2006.01)   *A61Q 11/00* (2006.01)
*A61K 8/02* (2006.01)   *A61K 8/41* (2006.01)
*A61K 8/49* (2006.01)

(52) Cooperative Patent Classification (CPC):
**A61K 8/26; A61K 8/0258; A61K 8/416;
A61K 8/4926; A61Q 11/00;** A61K 2800/412;
A61K 2800/524

(86) International application number:
**PCT/EP2021/066378**

(87) International publication number:
**WO 2021/259748 (30.12.2021 Gazette 2021/52)**

(54) **COMPOSITIONS COMPRISING ZINC AND ANTIMICROBIAL AGENT**

ZUSAMMENSETZUNGEN MIT ZINK UND ANTIMIKROBIELLEM MITTEL

COMPOSITIONS COMPRENANT DU ZINC ET AGENT ANTIMICROBIEN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.06.2020 EP 20181835**

(43) Date of publication of application:
**03.05.2023 Bulletin 2023/18**

(73) Proprietors:
• **Unilever IP Holdings B.V.
3013 AL Rotterdam (NL)**
Designated Contracting States:
**AL AT BE BG CH CZ DK EE ES FI FR GR HR HU IS
LI LT LU LV MC MK NL NO PL PT RO SE SI SK SM**
• **Unilever Global IP Limited
Wirral, Merseyside CH62 4ZD (GB)**
Designated Contracting States:
**CY DE GB IE IT MT RS TR**

(72) Inventors:
• **DABHOLKAR, Nandini, Sachin
Mumbai 400099 Andheri (IN)**
• **DASGUPTA, Anindya
Whitefield, Bangalore 560 066 (IN)**
• **KORANNE, Ketki, Yogesh
Mumbai 400099 Andheri (IN)**
• **SAJI, Maya, Treesa
Whitefield, Bangalore 560 066 (IN)**
• **SRIVASTAVA, Madalasa
Mumbai 400099 Andheri (IN)**
• **CHANDRASEKARAN, Sembian
Mumbai 400099 Andheri (IN)**

(74) Representative: **Tansley, Sally Elizabeth
Unilever Patent Group
Bronland 14
6708 WH Wageningen (NL)**

(56) References cited:
EP-A1- 2 105 469   WO-A1-2011/036031
WO-A1-2014/102032   WO-A1-2019/034352
WO-A1-2019/034387

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the Invention**

The present invention relates to personal care compositions in which the antimicrobial efficacy could manifest itself in e.g., inhibition of bacterial growth, malodour control or even extended or longer-lasting hygiene benefits.

The composition may be embodied in a variety of formats, such as a toothpaste.

**Background of the Invention**

A variety of microorganisms are present in our environment. They may be bacteria, viruses or even protozoans. While some microorganisms are useful to human beings, and some are commensals, a wide variety of microorganisms are undesired, but they inhabit many animate and inanimate surfaces that are of concern to human beings.

Usually such undesired microorganisms multiply quickly and their biological activity may manifest itself as e.g., malodour on certain animate surfaces and even inanimate surfaces. For example, when it comes to human skin, control of malodour assumes importance, for example in the underarm, on feet, as well as on the scalp, and hair.

Certain bacteria are known to cause malodour, example oral malodour.

Generally, malodour is caused by bacterial activity which creates certain malodourous compounds that are called as volatile sulphurous compounds such as hydrogen sulphide, methyl mercaptans, dimethyl sulphide, 3-mercapto-2-methyl-butan-1-ol and 3-mercapto-3-methylhexan-1-ol, ammonia and amines for example trimethylamine, putrescene (1,4-diaminobutane), indole, skatole (3-methyliondole), pyrazine; aldehydes, for example acrolein and butanal, alcohols for example oct-1-en-3-ol, ketones for example oct-1-en-3-one. On the other hand, malodourous compounds commonly found in sweat include volatile fatty acids (VFAs) and electrolytes such as isovaleric acid, 3-methyl-2-hexenoic acid and 4-methyloctanoic acid.

Malodor of the oral cavity is also known as halitosis or bad breath. It is a common non-therapeutic condition that can be mitigated by use of toothpastes or other cosmetic oral care compositions. In extreme cases, halitosis could become a therapeutic condition necessitating the use of prescription oral care products.

It is generally believed that the cause of this condition is due to anaerobic bacteria, especially gram-negative anaerobic bacteria, in the mouth. These bacteria generate volatile sulfur compounds (VSC's) which are known to cause oral or breath malodor. It is also recognized in the art that oral malodor not only comes from the posterior dorsal surface of the tongue but also from periodontal pockets. Furthermore, a person with gingivitis or periodontal disease may have increased oral malodor from disintegrated epithelial cells. Epithelial cells turn over faster if inflammation is present. Therefore, a larger number of these dead epithelial cells remain in the oral cavity and degrade into the malodorous compounds.functions of a dentifrice, a mouthwash and similar products include those of neutralising and/or removing unpleasant odours in the mouth and of making the user feel confident about freshness of the breath.

Dentifrice compositions like toothpastes, which are a type of personal care compositions, usually quickly neutralise and dislodge the odour-causing compounds and for a short period thereafter, do also mask remnants of any odorous compounds. Any enhancement in efficacy of such dentifrice compositions is highly desired.

The addition of substances having antibacterial/antimicrobial properties to toothpastes and mouthwashes, and the effect thereof, are well known. However, the effect of the active substances is only short-term, since the concentration of the substance in question drops rapidly following oral hygiene, and microbial development and bacterial growth set in again immediately. In this context it has been found that commercial oral care products display limited long-term effect in the oral cavity and that, for example, a large proportion of the active ingredients is removed from the mouth by rinsing with water after cleaning the teeth, for example, or by drinking or by consuming food. As a result of this, for example, oral malodour cannot be prevented over a prolonged period.

US2009010857 A (Unilever) discloses toothpastes comprising calcium carbonate as abrasive, water and zinc salt substantially all of which is in the form of water-solubilised zinc citrate.

WO17167535 A1 (Unilever) discloses toothpaste compositions having zinc salt for providing antimicrobial benefits, also having calcium-based abrasive, smectite clay and a buffering agent.

WO9939685 A1 (Unilever) discloses an oral care composition is provided in the form of a transparent gel, comprising an abrasive silica, a thickening silica, a polyol-humectant-containing liquid vehicle, and a low level of sodium bicarbonate. Despite the presence of the bicarbonate, the product is a transparent gel or white paste, and provides for a teeth-whitening effect, as well as a reduction of oral malodour, and neutralizes the acid, formed in the oral cavity by microbial decomposition of sugar. The composition may be coloured, and different compositions with different colours, e.g. a blue or a white one, may be dispensed from a single or a multicompartment container.

WO2003/002056 (Church And Dwight Co Inc) discloses an oral composition having antibacterial effective amount of cetylpyridinium chloride and guar hydroxypropyltrimonium chloride to bind to compounds which undesirably bind to cetylpyridinium chloride thereby enabling the cetylpyridinium chloride to effectively bind to tooth surfaces and perform an antibacterial function.

A bipolar clay based antimicrobial particle has been disclosed in US2012/0177712 A1 (Unilever). The precursor of the particle is an asymmetric 1:1 or 2:1:1 clay particle comprising alternating tetrahedral and octahedral sheets terminating with a tetrahedral sheet at one external surface plane and an octahedral sheet at another external surface plane with an antimicrobial group attached to the coordinating cation on one of the said external surface plane selected from a quaternary ammonium material comprising a single alkyl or alkenyl long chain having an average chain length greater than or equal to $C_{20}$, or a quaternary ammonium material.

WO14102032 A1 (Unilever) discloses oral care compositions having the bipolar antimicrobial particle as above and where the antibacterial agent is a quaternary ammonium material. The compositions control bacterial re-growth and provide immediate control on the bacterial count. The compositions comprise chalk or any calcium-based abrasive.

## Summary of the Invention

We have now surprisingly determined that the aforesaid bipolar antimicrobial particle interacts synergistically with a compound of zinc, such as zinc sulphate, to provide a variety of antimicrobial benefits inter alia to control or mitigate oral malodour.

Accordingly, this synergistic interaction can be provided to consumers in the form of a variety of personal care compositions.

In accordance with a first aspect is disclosed an oral care composition comprising:

(i) 0.1 to 10 wt% of a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antibacterial agent attached to the coordinating cation on one of the said external surface plane, where the antibacterial agent is a quaternary ammonium compound; and,

(ii) 0.001 to 10 wt% of a compound of zinc, wherein the median diameter of said particles (D50) is 0.1 to 10 $\mu$m, wherein said compound of zinc is zinc sulphate, zinc chloride, zinc nitrate, zinc citrate, zinc oxide, zinc lactate, zinc gluconate, zinc glycinate, zinc oleate, zinc hydroxide or zinc peroxide.

In accordance with a second aspect is disclosed non-therapeutic use of a composition of the first aspect in the form of an oral care composition for an improvement in oral hygiene.

In accordance with a third aspect is disclosed a non-therapeutic method of improving oral hygiene comprising a step of applying a composition of the first aspect in the form of an oral care composition in the oral cavity.

In accordance with a further aspect is disclosed a packaged oral care product comprising a composition of the first aspect in the form of an oral care composition in a package, preferably with a set of instructions to use the composition.

## Detailed Description

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

All references to the term/expression wt% or % by weight, shall mean percentage by weight of the composition, except where indicated otherwise.

"Oral care composition" refers to a composition for which the intended use can include oral care, oral hygiene, or oral appearance, or for which, the intended method of use can comprise administration to the oral cavity. In some embodiments, an oral care composition is not intentionally swallowed, but is rather retained in the oral cavity for a time sufficient to affect the intended utility. The oral care compositions as disclosed herein may be used in nonhuman mammals such as companion animals (e.g., dogs and cats), as well as by humans. In some embodiments, the oral care compositions as disclosed herein are used by humans.

Halitosis is the technical term for bad breath or malodour. It is common and may affect the quality of life.

Sometimes people who have bad breath may remain unaware. Oral malodor is caused by microbial action that leads to production of volatile sulfur compounds ("VSC") namely hydrogen sulphide, methylmercaptan and dimethyl sulphide. Therefore, treatment, which may be cosmetic (non-therapeutic) or may even be therapeutic, is often directed towards reduction of microorganisms inside the oral cavity or neutralization of the VSC compounds or by masking the malodour. A variety of oral care compositions available in a variety of formats are used for this purpose. The two most common formats or products are toothpastes and mouthwash. Such compositions usually contain one or more active ingredients included from e.g., 0.05 wt% to as high as 10 wt% of the composition. However, some actives may not reach the target site partly because they do not bind to the desired surface.

In some cases the active or actives may easily wash away and are therefore not retained for long periods in the oral cavity. Accordingly, there is a need for oral care compositions that allow that counteract malodour or reduce the malodour for extended period.

Oral malodour can be evaluated or measured by a variety of techniques. These include subjective assessments by human judges (organoleptic evaluations), industrial sulfide monitoring instruments which analyze exhaled air or microbial specimens removed from the mouth and gas chromatography of exhaled air, or bacterial specimens removed from the mouth. Another way is to identify hydrogen sulfide ($H_2S$)-producing bacteria among tongue biofilm microflora and to investigate the relationship between bacterial flora and $H_2S$ levels in mouth air. Oral malodour can be assessed by gas chromatography, and Breathtron and organoleptic scores., Another way is to use a portable sulfide monitor, the Halimeter®.

We have used soft tissue model to determine the efficacy of oral care compositions in accordance with this invention.

The procedure, is as follows:

Soft tissue model is an ex-vivo model that measures volatile sulphurous compounds generated by salivary bacteria. The method involves collection of early morning saliva from volunteers having good general and oral health. The saliva is pooled, oral epithelial cells are separated, sterilized and layered on the surface of agarose in a 12 well plate. Mixed salivary bacteria are then added in the plate. This is followed by treatment with the oral care composition, in this case, toothpaste samples. Cysteine-HCl is added post treatment as a precursor for volatile sulphurous compounds. Lead acetate paper is placed over the plate to act as an indicator of the volatile sulphurous compounds that are generated. The dark coloration of the lead acetate paper is recorded using a reflectometer in the form of L* a* b* values and the difference between the initial and final L* a* b* values [$\Delta E$], is used as a quantitative measure of the volatile sulphurous compounds so formed. Lower $\Delta E$ indicates a more efficacious composition.

$$\Delta E = \sqrt{(L_i - L_f)^2 + (a_i - a_f)^2 + (b_i - b_f)^2}$$

Preferably the data is statistically analysed using the Tukey-Kramer analysis comparing the mean measurements for the 3 repeats.

Formulation scientists have many options to choose from because a wide variety of active ingredients ranging from flavours to plant-based actives and to chemicals such as compounds of zinc. For longer-lasting activity, the usual approach is to increase the dosage of the ingredient concerned. However, that may lead to secondary problems like instability or may even affect some other desirable property of the compositions. For example, an excess of an ingredient may make the formulation unpalatable or too astringent. As the compositions of the invention can be personal care compositions, and particularly oral care compositions, a good mouthfeel would be preferred by consumers.

A variety of compounds of zinc are used in for example, oral care compositions. The most common ones are zinc sulphate and zinc citrate. The present applicant has disclosed oral care compositions that comprise bipolar antimicrobial particle that has been referred to earlier. The aforesaid materials each provide one or more technical effects as referred to earlier. However, we have now surprisingly observed that when used in combination, the two materials counteract malodour or reduce the malodour.

It is known that a set of technical features is regarded as a combination of features if the functional interaction between the features achieves a combined technical effect which is different from, e.g. greater than, the sum of the technical effects of the individual features. In other words, the interaction of the individual features produces a synergistic effect. This is different from a mere aggregation of features or properties.

It is believed that the bipolar antimicrobial particle and the compound of zinc interact synergistically to provide a combined effect that is not a simple additive effect.

Accordingly, in accordance with a first aspect is disclosed an oral care composition comprising:

(i) 0.1 to 10 wt% of a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antibacterial agent attached to the coordinating cation on one of the said external surface plane, where the antibacterial agent is a quaternary ammonium compound; and,

(ii) 0.001 to 10 wt% of a compound of zinc, wherein the median diameter of said particles (D50) is 0.1 to 10 $\mu$m, wherein said compound of zinc is zinc sulphate, zinc chloride, zinc nitrate, zinc citrate, zinc oxide, zinc lactate, zinc gluconate, zinc glycinate, zinc oleate, zinc hydroxide or zinc peroxide.

It is preferred that in the antimicrobial particle, said antibacterial agent is attached to coordinating cations on the external surface of the octahedral surface plane.

When the particles are of an increased size, the available surface area is believed to be lower. Accordingly, as the preferred particles have significantly lower size, is believed that more area is available for the antibacterial agent to form adduct therewith. Without further being bound by theory, it is believed that this makes more effective use of the antibacterial agent, thereby providing opportunity for substantial reduction in the dosage of the antibacterial agent. While conventional antibacterial agents need an optimised dosage of 0.2 to 0.5 wt%, the antibacterial agent, delivered in the form of the disclosed bipolar antibacterial particle, is seen to be highly effective even at an (actual) dosage of 0.001 %.

While not wishing to be further bound by theory it is believed that the preferred particles are appropriately sized to fit inside the interstitial spaces on the surface of the teeth which is believed to be a reason for prolonged efficacy. It is further believed that particles of lower size reduce, to a significant extent, any unfavorable interaction between the anionic surfactant and antibacterial agent even when the particle contains e.g., cetyl pyridinium chloride which is a quaternary ammonium compound and this is also believed to be a cause for longer lasting antibacterial benefits.

Particles of lower size also provide an effective mechanism for an increased loading of the antibacterial agent, if so desired.

Particle size distribution (D50) is also known as the median diameter or the medium value of the particle size distribution, it is the value of the particle diameter at 50 % in the cumulative distribution. It is an important parameter characterizing particle size. For example, if D50=5.8 $\mu$m, then 50 % of the particles in the sample are larger than 5.8 $\mu$m, and 50 % smaller than 5.8 $\mu$m. D50 is usually used to represent the particle size of group of particles. The D50 is the size in microns that splits the distribution with half above and half below this diameter.

The precursor of the antibacterial particle with bipolar topospecific characteristics is preferably an asymmetric 1:1 or 2:1:1 clay particle having alternating tetrahedral and an octahedral sheets terminating with a tetrahedral and an octahedral sheet at exterior surface planes. Particle of 1:1 clay is particularly preferred as the precursor. Preferred 1:1 clays include kaolinite and serpentine subgroups of minerals. The species included within the kaolinite subgroup include but are not limited to kaolinite, dickite, halloysite and nacrite. The species within the serpentine subgroup include but are not limited to chrysolite, lizardite, and amesite. Preferred 2:1:1clays include chlorite group of minerals. The chlorite comprises tetrahedral-octahedral-tetrahedral sheets like 2:1 clays, with an extra weakly bound brucite like layer between tetrahedral layers. The tetrahedral sheet preferably comprises coordinating tetrahedral cations of silicon. The tetrahedral sheet may also include isomorphously substituted coordinating tetrahedral cations which are not silicon. Isomorphously substituted coordinating tetrahedral cations include, but are not limited to, cations of aluminum, iron or boron.

The octahedral sheet preferably has coordinating octahedral cation of aluminum. The octahedral sheet may also comprise isomorphously substituted coordinating octahedral cations which are not aluminium. Isomorphously substituted coordinating octahedral cations include cations of magnesium or iron. It is preferred that the antimicrobial agent is attached to the coordinating cations on the exterior side of one of the external surface planes. Accordingly, the antimicrobial agent is attached to coordinating cations on the exterior side of the tetrahedral sheet. Alternatively, the antimicrobial molecule is attached to the coordinating cations on the exterior side of the octahedral sheet. Coordinating cations on the exterior side of each of the tetrahedral and the octahedral surface sheets are attached to a antimicrobial molecule, with the proviso that the antimicrobial molecule attached to the coordinating cations on the exterior side of the tetrahedral surface sheet is not identical to the molecule attached to the coordinating cations on the exterior side of the octahedral surface sheet. The antimicrobial agent is preferably attached to the coordinating cations on the external surface of the octahedral surface plane and is not preferably attached to coordination cations of non-exterior tetrahedral or octahedral plane or on the interior side of the surface sheets.

In the above mentioned particulate antimicrobial the clay:antibacterial ratio is from 1:0.001 to 1:1 more preferably from 1:0.001 and 1:0.1. It is preferred that in the antimicrobial particle, the antibacterial agent is attached to coordinating cations on the external surface of the octahedral surface plane.

Further preferably in every 100 parts of said bipolar antimicrobial particle comprises from 0.05 to 20 parts, more preferably 0.1 to 10 parts by weight of said antibacterial agent. The median diameter of said particles (D50) is 0.1 to 10 $\mu$m, preferably more preferably 0.4 to 1 $\mu$m and most preferably 0.5 to 0.8 $\mu$m.

It is further preferred that the quaternary ammonium material is at least one of cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide. More prererably the quaternary ammonium material is selected from cetylpyridinium chloride (CPC), cetyltrimethylammonium chloride (CTAC), cetyltrimethylammonium bromide (CTAB), benzalkonium chloride (BKC), benzethonium chloride, cetrimide or quaternium.

A particularly preferred antibacterial agent is Cetylpyridinium chloride. The antibacterial activity of cetylpyridinium chloride is, without being bound to the theory, believed to be linked to the cationic charge of its amine group. Thus, cetylpyridinium chloride is attracted to and binds to negatively-charged protein moieties on the cell membrane or cell wall of the microorganism and to tooth surfaces which are also typically negatively charged. The resulting attachment to microorganisms disrupts the cell wall structure causing leakage of the intracellular fluids, eventually killing the associated microorganism. However, cetylpyridinium chloride is generally not effective in many systems because of its tendency to complex with component that carry a negative charge. When bound to the clay in this manner, cetylpyridinium chloride is unavailable for effective contact with tooth surfaces and microorganisms, thereby rendering the active agent ineffective for its intended purpose.

For this reason, cetylpyridinium chloride has not been totally effective in typical oral care products for the treatment and/or prevention of gingivitis, plaque, periodontal disease, and/or breath malodor. For example, toothpaste compositions typically include anionic surfactants and artificial sweetening agents. These components of toothpaste compositions typically bind to cetylpyridinium chloride and thereby render it ineffective or substantially less effective as an antibacterial agent. Other components typically found in a toothpaste composition such as abrasives also bind to cetylpyridinium chloride. Accordingly, the use of cetylpyridinium chloride in toothpaste compositions has been problematic. Even in commercial mouthwash products that contain cetylpyridinium chloride, the availability of cetylpyridinium chloride at tooth surfaces is very low and therefore its antibacterial effectiveness is limited.

Further details of the particle may be found in WO2011/036031A1 (Unilever).

The compound of zinc

It is preferred that solubility of the compound of zinc at 20°C is 0.001 to 80 g/100 g water. Insoluble and soluble zinc salts may be used, alone or in combination, although the use of soluble zinc salts is preferred (zinc salts with aqueous solubility greater than 0.01 g/100g water).

The compositions comprise at least one of zinc sulphate, zinc chloride, zinc nitrate, zinc citrate, zinc oxide, zinc lactate, zinc gluconate, zinc glycinate, zinc oleate, zinc hydroxide or zinc peroxide. In some embodiments, a mixture of zinc sulphate and zinc citrate is preferred. In other embodiments, a mixture of zinc oxide and zinc sulphate is preferred. It is preferred that the composition is a toothpaste, toothpowder, breath-freshening spray, breath-freshening strip, breath freshening film, or a mouthwash.

More preferably the composition is a toothpaste. Alternatively, and more preferably the composition is a mouthwash.

One of the functions of a mouthwash and similar products is to remove unpleasant mouth odours and of making the user feel confident that their breath will not offend. However there remains a need to further mitigate mouth odours, especially from mouthwashes that are marketed as having mild formulations. Mild formulations contain low levels/no alcohol and no or low levels of anionic surfactants. The term "mouthwash" generally denotes liquid formulations which are used to rinse the surfaces of the oral cavity and provide the user with a sensation of oral cleanliness and refreshment. The mouthwash is an oral care composition that is not intentionally swallowed for purposes of systemic administration of therapeutic agents, but is applied to the oral cavity, used to treat the oral cavity and then expectorated.

Other Ingredients

When the composition of the invention is an oral care composition it preferably comprises one or more of the ingredients discussed hereinafter. The exact ingredients and the dosage (wt%) would depend on the nature and type of the oral care composition. For example, while a toothpaste invariably comprises an abrasive, a mouthwash does not need any abrasive.

When said composition is a toothpaste, it preferably comprises 20 to 60 wt% of one or more humectants.

Humectants are generally included in toothpastes for a soft a supple mouth feel. Humectants also reduce the tendency of toothpastes to lose moisture. It is preferred that the humectant is at least one of glycerine, sorbitol, maltitol and xylitol. Sorbitol is available as a 70% aqueous solution. Preferably the compositions comprise glycerine and sorbitol for a lubricated mouth feel, but their cumulative levels do not exceed the disclosed upper limit. Lower humectant content provides an effective way to reduce the cost of the product.

When the compositions of the invention is a toothpaste it preferably comprises an abrasive. The primary function of cleaning agents or abrasives is to provide the necessary cleaning and stain removal to the toothpaste formulation. Typically, cleaning is directly correlated to the abrasivity of silica on tooth dentin and measured by RDA. In other words, the higher the RDA, the higher the abrasivity. Even though this is typically true, some of our cleaning grades can provide high cleaning efficiency at reduced level of abrasion when compared with competitive cleaning grades.

In one aspect the abrasive is silica. Such toothpastes are usually transparent or translucent gels. The term gel indicates physical appearance of the compositions. The gel compositions are transparent to light, i.e. they allow visible light to pass through. Clear gel toothpastes are popular with consumers. When the oral care composition of the invention is a toothpaste in the form of a gel, it is preferred that the compositions do not comprise more than 5 wt%, preferably more than 2 wt% and more preferably more than 1 wt% calcium-based abrasive. That might affect the transparent nature of the compositions.

On the other hand, there are some other types of dentifrice compositions that are not gels but are opaque. That is because such compositions usually contain opacifying ingredients such as chalk or other calcium-based abrasives.

Preferably the composition comprises 5 to 20 wt% silica. Further preferably the composition the silica consists of medium- and high-abrasive silica, where medium-abrasive silica is in majority. Their relative proportions may vary. Preferably the abrasive silica is low refractive index silica with an apparent refractive index in the range of 1.41 to 1.47, preferably 1.435 to 1.445, preferably having a weight mean particle size of between 5 and 15 $\mu$m, a BET (nitrogen) surface area of between 10 and 100 $m^2$/g and an oil absorption of about 70 to 150 $cm^3$/100 g. Examples of suitable low refractive index abrasive silicas having an R.I. of between 1.435 and 1.445 are Tixosil 63 and 73 ex Rhone Poulenc; Sident 10 ex Degussa; Zeodent 113 ex Zeofinn; Sorbosil AC 77 ex Ineos having an R.I. of approximately 1.440. Other examples include the Sylodent® and Syloblanc® range of abrasive silicas from Grace.

Further preferably the oral care compositions comprise 2 to 15 wt% thickening silica. The primary function of thickening silica is to build viscosity, without increasing the level of abrasion (RDA) in the overall formulation. Also, they can be used as texturing agents in formulations that use alternative abrasives other than silica. Our grades of thickening silicas can be categorized by their physical characteristics. These physical characteristics have effect on the rheological properties of the formulation. For example, and perhaps more relevantly, the higher the oil absorption of the thickener, the higher the viscosity imparted to the formulation. In addition to having a substantial impact on the viscosity of the toothpaste system, the thickening silica also plays a role in the mouthfeel of the paste. If the level of the cleaning agent in the system is low, than the formulator may wish to use a less efficient thickener to achieve an overall solids level consistent with the desired mouthfeel. In general, the paste formulation should include at least 15% up to 30% total silica concentration. The split of this percentage between thickener and cleaning agent depends on the choice of silicas, desired cleaning level, etc.

This is especially the case when the compositions comprise silica as the abrasive. A wide variety of thickening silicas are commercially available to choose from. When present, preferred thickening silicas include AEROSIL®T series from Degussa or the CAB-O-SIL® series from Cabot Corporation, silica gels such as the SYLODENT® or SYLOX® series from W. R.Grace & Co or precipitated silica such as ZEOTHIX® 265 from J. M. Huber Corporation. Useful silica thickeners also include ZEODENT® 165, ZEODENT® 163 and/or 167 and ZEOFREE® 153, 177, and/or 265 silicas, all available from J. M. Huber Corporation. Other preferred thickening silicas include MFIL®, MFIL®-P (From Madhu Silica), SIDENT® 22 S and AEROSIL® 200 (Ex. Evonik Industries), SYLODENT® and PERKASIL® thickening silicas from WR Grace & Company and Tixosil® 43 and 331 from Rhodia, synthetic finely divided pyrogenic silica such as those sold under the trademarks SYLOID® 244, SYLOID® 266 and AEROSIL® D-200.

Alternatively, the oral care compositions of the invention comprise calcium -based abrasive. It is particularly preferred abrasive is fine ground natural chalk (FGNC). It is obtained from limestone or marble. FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion.

Other preferred calcium-based abrasives include dicalcium phosphate (DCP), calcium pyrophosphate and precipitated calcium carbonate (PCC). When a combination of Calcium-based abrasives is used, it is preferred that FGNC is 35 to 100 %, more preferably 75 to 100 % and especially from 95 to 100 % of the total abrasive. In such cases, the balance, most preferably, is PCC.

Other abrasives can also be used depending upon the intended degree of abrasion and the composition of the paste. These include synthetic abrasive polishing agents such as amorphous precipitated silica and silica gels. Other abrasives include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

A mouthwash composition according preferably comprises an aqueous continuous phase. The amount of water generally ranges from 70 to 99 wt%. A mouthwash composition preferably comprises further ingredients to enhance performance and/or consumer acceptability, such as the humectants and surfactants. The amount of humectant generally ranges from 4 to 20 percent, more preferably from 7 to 15 percent by weight based on the total weight of the mouthwash.

Preferred humectants that are present at the above levels include polyols, particularly preferred is sorbitol.

Surfactants

Oral care compositions, especially toothpastes generally contain a surfactant, also commonly referred to as sudsing agent. Suitable surfactants are those which are reasonably stable and provide foam throughout a wider pH range. It is preferred that compositions comprise an anionic surfactant.

Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type.

Preferably the oral care compositions comprise 0.25 to 12 wt%, more preferably from 0.5 to 8 wt%, and most preferably from 1 to about 6 wt% anionic surfactants. Some anionic surfactants, in particular, sodium lauryl sulphate itself have antibacterial effect. Such action provides some degree of instant antibacterial effect. However, this effect is generally very short-lived. Other surfactants like nonionic, amphoteric or zwitterionic surfactants may also be included.

Nonionic surfactants can be broadly defined as compounds produced by the condensation of alkylene oxide groups (hydrophilic in nature) with an organic hydrophobic compound which may be aliphatic or alkyl- aromatic in nature. Examples of suitable nonionic surfactants include poloxamers (sold under trade name PLURONIC®), polyoxyethylene, polyoxyethylene sorbitan esters (sold under trade name TWEENS®), POLYOXYL® 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials.

Further preferably the oral care compositions of the invention comprises a thickening system which comprises thickening silica and at least one of carboxymethyl cellulose, xanthan gum or guar gum. Other binders and thickeners such as sodium carboxymethylcellulose, xanthan gum, gum arabic may also be included, as well as synthetic polymers such as polyacrylates and carboxyvinyl polymers such as Carbopol®. It is preferred that dentifrice compositions of the invention comprise a thickening system which comprises thickening silica and at least one of carboxymethyl cellulose, xanthan gum or guar gum.

Preferred oral care compositions comprise a binder, which lends a good structure to the paste. Cellulosic binders are especially preferred. Preferred cellulosic binders include cellulose ethers, which include hydroxyethyl cellulose (HEC), hydroxypropyl cellulose (HPC), ethylhydroxyethyl cellulose (EHEC), carboxymethyl cellulose (CMC), carboxymethylhydroxyethyl cellulose (CMHEC), hydroxypropylhydroxyethyl cellulose (HPHEC), methyl cellulose (MC), methylhydroxypropyl cellulose (MHPC), methylhydroxyethyl cellulose (MHEC), carboxymethylmethyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethylhydroxyethyl cellulose (HMEHEC), hydrophobically modified carboxymethylhydroxyethyl cellulose (HMCMHEC), hydrophobically modified hydroxypropylhydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC).

Other cellulosic binders include cationic hydroxyethyl cellulose (cationic HEC), cationic hydrophobically modified hydroxyethyl cellulose (cationic HMHEC) and microcrystalline cellulose.

A highly preferred binder is Sodium carboxymethyl cellulose (SCMC). Particularly preferred sodium carboxymethyl celluloses include those with degree of substitution of from 0.6 to 0.99, preferably from 0.7 to 0.95.

Preferred toothpaste compositions may also include one or more other thickening agents such as carboxyvinyl polymers which include carbomers which are commercially available from B. F. Goodrich as the CARBOPOL® series, including CARBOPOL® 934, 940, 941 and 956.

Other preferred grades include acrylates/$C_{10-30}$ alkyl acrylate crosspolymers which are commercially available as ULTREZ® 21, PEMULEN® TR-1, and PEMULEN®TR-2, from Noveon Corporation. Preferred compositions can include 0.05 to 10 wt%, more preferably 0.1 to 5 wt%, and even more preferably 0.25 to about 4 wt% of other thickening agents.

The oral care compositions of the invention may contain optional further ingredients such as cosmetically acceptable carriers like starch or sucrose.

Preferred compositions can have alkali metal silicate. The alkali metal is sodium or potassium, preferably sodium. Sodium silicate is generally available as 10 to 40 % aqueous solution, most common being 30 % solution. Sodium silicate is available as neutral sodium silicate or alkaline sodium silicate. Preferred toothpastes have neutral sodium silicate. Sodium silicate is available with varying ratios of $Na_2O$: $SiO_2$.

Sodium silicate with $Na_2O$: $SiO_2$ ratio in the range of 3.0 to 3.8 is preferred, more highly preferred range being 3.25 to 3.5. Preferred toothpastes include 0.1 to 5 wt% silicate (on dry weight basis). Thus, a 30 % solution of sodium silicate is added to the composition in an amount in the range of 0.3 to 16 wt%.

Flavours such as peppermint and spearmint oils may also be included, as well as preservatives, colouring agents, pH adjusting agents, sweetening agents and so on. Suitable flavoring components include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants are the paramenthane carboxyamide agents such as N-ethyl-p-menthan-3-carboxamide (known commercially as "WS-3®") and mixtures thereof. The flavour is generally from 0.001 to 5 wt%.

Anti-caries agents such as sodium- and stannous fluoride, aminefluorides, monosodiumfluorophosphate, casein, plaque buffers such as urea, pyruvates, arginine, small peptides, calcium lactate, calcium glycerophosphate, strontium polyacrylates may also be included. A preferred anti-caries agent is sodium monofluorophosphate. Other optional ingredients include vitamins such as Vitamin C, and plant extracts.

Desensitising agents such as potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral care compositions may comprise anti-calculus agents such a alkali metal pyrophosphates, hypophosphite-containing polymers, organic phosphonates and phosphor-citrates.

Other optional ingredients that may be included are e.g. bleaching agents such as peroxy compounds e.g. potassium peroxy diphosphate, effervescing systems such as sodium bicarbonate/citric acid systems and colour change systems.

The compositions of the invention may also contain coloured particles suspended therein, e.g. coloured silica agglomerates or other coloured particles to impart a "speckled" appearance to the dentifrices. The oral care compositions may also contain stripes of a coloured paste, to provide for a visually clear gel-type dentifrice with coloured stripes or a separate coloured phase

Toothpastes with calcium containing abrasives especially chalk are prone to bacterial growth. Certain preservatives, e.g. methyl, ethyl, butyl, propyl and isopropyl esters of parahydroxybenzoic acid may be particularly useful against bacterial growth. A mixture of methyl, ethyl, butyl and propyl esters of parahydroxybenzoic acid is particularly preferred.

The activity of this mixture can be enhanced by adding phenoxyethanol. Formaldehyde and dimethyl hydantoin are other preferred preservatives. Preservatives are generally included at 0.005 to 0.8 wt%.

The oral care composition of the present invention is prepared by conventional methods of making oral care compositions. Such methods include mixing the ingredients under moderate shear and atmospheric pressure.

Typically, the composition is packaged.

In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out one to five times monthly.

In some embodiments, the methods comprise the step of rinsing the oral cavity with a composition as described herein. In some embodiments, 5 ml or more of the composition is gargled. In some embodiments, 10 ml or more is used. In some embodiments, 10-50 ml is used. In some embodiments, 15-25 ml or more is used. In some embodiments, 15 ml or more is used. In some embodiments, the individual gargles with the composition multiple times per day. In some embodiments, the individual gargles with the composition on multiple days. In some embodiments, the individual gargles with the composition every 4 to 6 hours up to 6 times per day.

In accordance with a second aspect is disclosed non-therapeutic use of a composition of the first aspect in the form of an oral care composition for treatment of bad breath.

It is preferred that said oral hygiene is improved at least by a reduction in oral malodour of the user. Preferably the oral malodour is caused by the activity of salivary bacteria which generate volatile sulphurous compounds on at least the soft tissues inside said oral cavity. Further preferably this malodour is reduced due to decrease in said volatile sulphurous compounds. In one aspect the use is for non-therapeutic purpose. Alternatively the use is for therapeutic purpose.

Therapeutic treatments can be summarized as being measures directed to the maintenance (prophlyaxis) or restoration (therapy) of health. 'Therapy' is concerned with bringing a body from a pathological state back to its normal, healthy state and with preventing a pathological state. Treatment by therapy is defined as any treatment which is designed to cure, alleviate, remove or lessen the symptoms of, or prevent or reduce the possibility of contracting any disorder or malfunction of the human or animal body.

In accordance with a third aspect is disclosed a non-therapeutic method of treating bad breath comprising a step of applying a composition of the first aspect in the form of an oral care composition in the oral cavity.

In accordance with a further aspect is disclosed a composition for use as a medicament in the treatment of halitosis or bad breath comprising:

(i) 0.1 to 10 wt% of a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antibacterial agent attached to the coordinating cation on one of the said external surface plane, where the antibacterial agent is a quaternary ammonium compound; and,

(ii) 0.001 to 10 wt% of a compound of zinc, wherein the median diameter of said particles (D50) is 0.1 to 10 $\mu$m

Preferably the use of the composition as above brings about at least 30% reduction in at least one volatile sulphurous compound present in the oral cavity. Further preferably the reduction is from 30 % to 90%. The volatile sulphurous compound is preferably at least one of hydrogen sulfide, methyl mercaptan, or dimethyl sulfide.

In accordance with a further aspect is disclosed a packaged oral care product comprising an oral care composition of the first aspect in a package, preferably with a set of instructions to use the composition.

The invention will now be further described with reference to the following non-limiting examples.

## EXAMPLES

Example-1: Oral care composition - Formulation and Testing

A series of toothpastes were prepared. Details are included in Table 1. Composition 1, 2 and 3 were outside the scope of the invention, PL indicates placebo formulation. The compositions Ex1 and Ex2 were within the scope of the invention.

All the formulation of Table 1 including the placebo were subjected to malodour evaluation using the Soft Tissue Model described earlier. The observations, including statistical significance according to Anova and Tukey Tests are also summarised in the table.

**Table 1**

| Ingredients | Composition/Reference number and wt% of ingredients | | | | | |
|---|---|---|---|---|---|---|
| | PL | 1 | 2 | 3 | Ex1 | Ex2 |
| Sorbitol (70%) | 58.0 | 58.0 | 58.0 | 58.0 | 58.0 | 58.0 |
| Thickening Silica | 8.5 | 8.5 | 8.5 | 8.5 | 8.5 | 8.5 |
| Abrasive Silica (med) | 9.0 | 9.0 | 9.0 | 9.0 | 9.0 | 9.0 |
| Abrasive Silica (high) | 1.6 | 1.6 | 1.6 | 1.6 | 1.6 | 1.6 |
| SLS | 2.7 | 2.7 | 2.7 | 2.7 | 2.7 | 2.7 |
| Zinc sulphate | -- | 0.2 | -- | -- | 0.2 | 0.2 |
| Particle** | -- | -- | 1.0 | 1.5 | 0.5 | 1.0 |
| SCMC 9H | 0.7 | 0.7 | 0.7 | 0.7 | 0.7 | 0.7 |
| NaOH solution (10%) | 0.3 | 0.3 | 0.3 | 0.3 | 0.3 | 0.3 |
| Water and others | 100.0 | 100.0 | 100.0 | 100.0 | 100.0 | 100.0 |
| ΔE | 52.79 | 35.57 | 20.49 | 19.14 | 10.66 | 9.57 |

(continued)

| Ingredients | Composition/Reference number and wt% of ingredients | | | | | |
|---|---|---|---|---|---|---|
| | PL | 1 | 2 | 3 | Ex1 | Ex2 |
| Statistical significance | B | C | E | E | F | F |
| Note*: the particle had 2.7% cetyl pyridinium chloride on kaolin. Therefore the composition of the particle was 2.7 wt% cetyl pyridnium chloride and 97.3 wt% kaolin. For example, in Ex2 the effective amount of quaternary ammonium compound was 0.027 wt% in the toothpaste formulation. D50 of the particles was 0.5 $\mu$m. | | | | | | |

As far as the statistical significance is concerned, as the tests were paired-tests, compositions not connected by the same letter are significantly different.

As $\Delta$E signifies reduction in malodour, it is inversely proportional to the efficacy of the composition. In other words, a composition of lower $\Delta$E is more efficacious than a composition with higher $\Delta$E.

Therefore, the data in Table 1 indicates clearly that Ex1 and Ex2 brought about a statistically significant improvement in oral hygiene as compared to the placebo. the data in Table 1 also indicates clearly that Ex1 and Ex2 brought about a significant improvement in oral hygiene as compared to compositions 1,2 and 3 which were comparative compositions outside the scope of the invention. The oral hygiene is improved reduction in oral malodour of the user. According to the procedure, the malodour is caused by the activity of salivary bacteria which generate volatile sulphurous compounds on at least the soft tissues (tongue) inside said oral cavity. It is evidently clear that use of the composition according to the invention reduces oral malodour by decreasing the volatile sulphurous compounds.

The exemplified examples clearly indicate that the bipolar antimicrobial particle interacts synergistically with a compound of zinc, (in the example, zinc sulphate) to provide a variety of antimicrobial benefits inter alia to control or mitigate malodour, especially oral malodour. Accordingly, this synergistic interaction can be utilised and provided to consumers in a variety of home care and personal care compositions.

**Claims**

1.  An oral care composition comprising:

    i) 0.1 to 10 wt% of a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antibacterial agent attached to the coordinating cation on one of the said external surface plane, where the antibacterial agent is a quaternary ammonium compound; and,
    ii) 0.001 to 10 wt% of a compound of zinc, wherein the median diameter of said particles (D50) is 0.1 to 10 $\mu$m, wherein said compound of zinc is zinc sulphate, zinc chloride, zinc nitrate, zinc citrate, zinc oxide, zinc lactate, zinc gluconate, zinc glycinate, zinc oleate, zinc hydroxide or zinc peroxide.

2.  An oral care composition as claimed in claim 1 wherein said quaternary ammonium material is at least one of cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride or domiphen bromide.

3.  An oral care composition as claimed in any one of claims 1 to 2 wherein solubility of said compound of zinc at 20°C is 0.001 to 80 g/100 g water.

4.  An oral care composition as claimed in any of claims 1 to 3 comprising 0.1 to 10 wt% bipolar antimicrobial particle.

5.  Non-therapeutic use of an oral care composition as claimed in any of claims 1 to 4 for treatment of bad breath.

6.  A non-therapeutic method of treating bad breath comprising a step of applying an oral care composition as claimed in any of claims 1 to 4 in the oral cavity.

7.  An oral care composition for use as a medicament in the treatment of halitosis or bad breath, comprising:

EP 4 171 478 B1

i) 0.1 to 10 wt% of a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antibacterial agent attached to the coordinating cation on one of the said external surface plane, where the antibacterial agent is a quaternary ammonium compound; and,
ii) 0.001 to 10 wt% of a compound of zinc, wherein the median diameter of said particles (D50) is 0.1 to 10 $\mu$m, wherein said compound of zinc is zinc sulphate, zinc chloride, zinc nitrate, zinc citrate, zinc oxide, zinc lactate, zinc gluconate, zinc glycinate, zinc oleate, zinc hydroxide or zinc peroxide.

**Patentansprüche**

1. Mundpflegezusammensetzung, umfassend:

    i) 0,1 bis 10 Gew.-% eines bipolaren antimikrobiellen Partikels, dessen Vorläufer ein asymmetrisches 1:1- oder 2:1:1-Tonpartikel ist, das alternierend tetraedrische und oktaedrische Schichten umfasst, die mit einer tetraedrischen Schicht auf einer äußeren Oberflächenebene und einer oktaedrischen Schicht auf einer anderen äußeren Oberflächenebene enden, wobei ein antibakterielles Mittel an das koordinierende Kation auf einer der äußeren Oberflächenebenen gebunden ist, wobei das antibakterielle Mittel eine quaternäre Ammoniumverbindung ist; und
    ii) 0,001 bis 10 Gew.-% einer Zinkverbindung, wobei der mittlere Durchmesser der Partikel (D50) 0,1 bis 10 $\mu$m beträgt, wobei die Zinkverbindung Zinksulfat, Zinkchlorid, Zinknitrat, Zinkcitrat, Zinkoxid, Zinklaktat, Zinkglukonat, Zinkglycinat, Zinkoleat, Zinkhydroxid oder Zinkperoxid ist.

2. Mundpflegezusammensetzung, wie im Anspruch 1 beansprucht, wobei das quaternäre Ammoniummaterial mindestens eines der folgenden Substanzen ist: Cetylpyridiniumchlorid (CPC), Cetyltrimethylammoniumchlorid (CTAC), Cetyltrimethylammoniumbromid (CTAB), Benzalkoniumchlorid (BKC), Benzethoniumchlorid, Cetrimid, Quaternium, Tetrabutylammoniumbromid, Undecylenamido-propyltrimoniummethosulfat, Methylbenzethoniumchlorid, Cetethyldimoniumbromid, Cetromoniumtosylat, Cocotrimoniumchlorid, Dodecylbenzyltrimoniumchlorid, Laurylisochinoliumbromid, Laurylpyridiniumchlorid, Dequaliniumchlorid oder Domiphenbromid.

3. Mundpflegezusammensetzung, wie in irgendeinem der Ansprüche 1 bis 2 beansprucht, wobei die Löslichkeit der Zinkverbindung bei 20°C 0,001 bis 80 g/ 100 g Wasser beträgt.

4. Mundpflegezusammensetzung, wie in einem der Ansprüche 1 bis 3 beansprucht, umfassend 0,1 bis 10 Gew.-% bipolares antimikrobielles Partikel.

5. Nicht-therapeutische Verwendung einer Mundpflegezusammensetzung, wie in einem der Ansprüche 1 bis 4 beansprucht, zur Behandlung von schlechtem Atem.

6. Nicht-therapeutisches Verfahren zur Behandlung von schlechtem Atem, umfassend den Schritt des Auftragens einer Mundpflegezusammensetzung, wie in einem der Ansprüche 1 bis 4 beansprucht, in die Mundhöhle.

7. Mundpflegezusammensetzung zur Verwendung als Medikament bei der Behandlung von Halitosis oder schlechtem Atem, umfassend:

    i) 0,1 bis 10 Gew.-% eines bipolaren antimikrobiellen Partikels, dessen Vorläufer ein asymmetrisches 1:1- oder 2:1:1-Tonpartikel ist, das alternierend tetraedrische und oktaedrische Schichten umfasst, die mit einer tetraedrischen Schicht auf einer äußeren Oberflächenebene und einer oktaedrischen Schicht auf einer anderen äußeren Oberflächenebene enden, wobei ein antibakterielles Mittel an das koordinierende Kation auf einer der äußeren Oberflächenebenen gebunden ist, wobei das antibakterielle Mittel eine quaternäre Ammoniumverbindung ist; und
    ii) 0,001 bis 10 Gew.-% einer Zinkverbindung, wobei der mittlere Durchmesser der Partikel (D50) 0,1 bis 10 $\mu$m beträgt, wobei die Zinkverbindung Zinksulfat, Zinkchlorid, Zinknitrat, Zinkcitrat, Zinkoxid, Zinklaktat, Zinkglukonat, Zinkglycinat, Zinkoleat, Zinkhydroxid oder Zinkperoxid ist.

**Revendications**

1. Composition d'hygiène buccale comprenant :

   i) 0,1 à 10 % en poids d'une particule antimicrobienne bipolaire dont le précurseur est une particule d'argile asymétrique 1/1 ou 2/1/1 comprenant des feuillets tétraédriques et octaédriques alternés se terminant par un feuillet tétraédrique au niveau d'un plan de surface externe et un feuillet octaédrique au niveau d'un autre plan de surface externe avec un agent antibactérien attaché au cation de coordination sur l'un desdits plans de surface externes, où l'agent antibactérien est un composé d'ammonium quaternaire ; et
   ii) 0,001 à 10 % en poids d'un composé du zinc,

   dans laquelle le diamètre médian desdites particules (D50) est de 0,1 à 10 $\mu$m, dans laquelle ledit composé du zinc est le sulfate de zinc, le chlorure de zinc, le nitrate de zinc, le citrate de zinc, l'oxyde de zinc, le lactate de zinc, le gluconate de zinc, le glycinate de zinc, l'oléate de zinc, l'hydroxyde de zinc ou le peroxyde de zinc.

2. Composition d'hygiène buccale selon la revendication 1, dans laquelle ledit matériau d'ammonium quaternaire est au moins l'un parmi le chlorure de cétylpyridinium (CPC), le chlorure de cétyltriméthylammonium (CTAC), le bromure de cétyltriméthylammonium (CTAB), le chlorure de benzalkonium (BKC), le chlorure de benzéthonium, le cétrimide, le quaternium, le bromure de tétrabutylammonium, le méthosulfate d'undécylène-amidopropyltrimonium, le chlorure de méthylbenzéthonium, le bromure de cététhyldimonium, le tosylate de cétromonium, le chlorure de (coprah-yl) trimonium, le chlorure de dodécylbenzyltrimonium, le bromure de laurylisoquinolium, le chlorure de laurylpyridinium, le chlorure de déqualinium et le bromure de domiphène.

3. Composition d'hygiène buccale selon l'une quelconque des revendications 1 et 2, dans laquelle la solubilité dudit composé du zinc à 20°C est de 0,001 à 80 g/100 g d'eau.

4. Composition d'hygiène buccale selon l'une quelconque des revendications 1 à 3, comprenant 0,1 à 10 % en poids de particule antimicrobienne bipolaire.

5. Usage non thérapeutique d'une composition d'hygiène buccale selon l'une quelconque des revendications 1 à 4 pour le traitement de la mauvaise haleine.

6. Méthode non thérapeutique de traitement de la mauvaise haleine, comprenant une étape d'application d'une composition d'hygiène buccale selon l'une quelconque des revendications 1 à 4 dans la cavité orale.

7. Composition d'hygiène buccale pour une utilisation en tant que médicament dans le traitement de l'halitose ou de la mauvaise haleine, comprenant :

   i) 0,1 à 10 % en poids d'une particule antimicrobienne bipolaire dont le précurseur est une particule d'argile asymétrique 1/1 ou 2/1/1 comprenant des feuillets tétraédriques et octaédriques alternés se terminant par un feuillet tétraédrique au niveau d'un plan de surface externe et un feuillet octaédrique au niveau d'un autre plan de surface externe avec un agent antibactérien attaché au cation de coordination sur l'un desdits plans de surface externes, où l'agent antibactérien est un composé d'ammonium quaternaire ; et
   ii) 0,001 à 10 % en poids d'un composé du zinc,

   dans laquelle le diamètre médian desdites particules (D50) est de 0,1 à 10 $\mu$m, dans laquelle ledit composé du zinc est le sulfate de zinc, le chlorure de zinc, le nitrate de zinc, le citrate de zinc, l'oxyde de zinc, le lactate de zinc, le gluconate de zinc, le glycinate de zinc, l'oléate de zinc, l'hydroxyde de zinc ou le peroxyde de zinc.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009010857 A, Unilever
- WO 17167535 A1, Unilever
- WO 9939685 A1, Unilever
- WO 2003002056 A, Church And Dwight Co Inc
- US 20120177712 A1, Unilever
- WO 14102032 A1, Unilever
- WO 2011036031 A1, Unilever